(19) Europäisches Patentamt
European Patent Office
Office européen des brevets

(11) **EP 4 491 128 A1**

(12) **EUROPEAN PATENT APPLICATION**

(43) Date of publication:
**15.01.2025 Bulletin 2025/03**

(21) Application number: **23315279.2**

(22) Date of filing: **12.07.2023**

(51) International Patent Classification (IPC):
***A61B 8/08*** *(2006.01)* ***A61B 8/00*** *(2006.01)*

(52) Cooperative Patent Classification (CPC):
**A61B 8/4218; A61B 6/487; A61B 8/085; A61B 8/145; A61B 8/483; A61B 8/54; A61B 34/10; G06N 20/00;** A61B 6/504; A61B 8/0891; A61B 8/10; A61B 8/4254; A61B 8/4263; A61B 8/429; A61B 8/4411; (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Caranx Medical**
**75013 Paris (FR)**

(72) Inventors:
- **AGNUS, Vincent**
**67400 Illkirch-Graffenstaden (FR)**
- **BERTHET-RAYNE, Pierre**
**06800 Cagnes-sur-Mer (FR)**
- **BECHAR, Ikhlef**
**06410 Biot (FR)**
- **CERRUTI, Giulio**
**06700 Saint-Laurent-Du-Var (FR)**
- **NAAMANI, Belinda Meriem**
**34000 Montpellier (FR)**
- **SMITS, Jonas Victor Harmen**
**3360 Bierbeek (BE)**

(74) Representative: **Hepp Wenger Ryffel AG**
**Friedtalweg 5**
**9500 Wil (CH)**

# (54) A COMPUTER IMPLEMENTED METHOD, A ROBOTIC SYSTEM, AND A COMPUT-ER PROGRAM PRODUCT FOR DETERMINING AN ACCESS SITE FOR AN INTRAVASCULAR ACCESS FOR SURGICAL INTERVENTION

(57) The invention relates to a computer implemented method for determining an access site (1) for an intravascular access for a surgical intervention, preferably executed on at least one control unit (2), the method comprising the steps: receiving two- or three-dimensional image data (3), positioning an imaging probe (5) by actuation of a positioning module (6) in a first orientation (51) based on the image data (3), moving the imaging probe (5) by actuation of the positioning module (6) based on the image data (3) in the first orientation (51) along the longitudinal axis (L) of the elongated bodily structure (4), analysing the received two- or three-dimensional image data in view of the presence of a landmark (7) of the elongated bodily structure (4), if no landmark (7) is detected repeating the prior steps, if a landmark (7) is detected based on the received image data (3) positioning the imaging probe (5) by actuation of the positioning module (6) based on the image data (3) from the first orientation (51) to a second orientation (52), and determining the access site (1) based on the image data (3) in the second orientation (52).

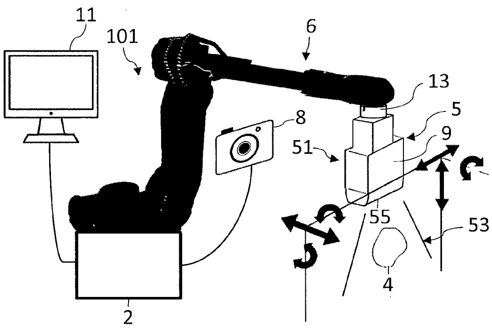

Fig. 2

EP 4 491 128 A1

(52) Cooperative Patent Classification (CPC): (Cont.)
A61B 8/4477; A61B 8/4488; A61B 8/5261;
A61B 2034/102; A61B 2090/378; A61M 5/427

## Description

The invention relates to a computer implemented method, a robotic system, and a computer program product for determining an access site for an intravascular access for a surgical intervention according to the independent claims.

Endovascular access, in particular transfemoral access, for performing surgical endovascular interventions can be associated with a plurality of vascular complications, such as bleeding, plaque detachment, increased morbidity/mortality, high follow-up costs, and/or long-term impairment of the condition of the patient.

Access site determination in the prior art is based on vascular imaging and relies on the clinician's ability to align an imaging probe and medical instrument based on a patient's anatomy. However, this approach has a number of limitations and may result in prolonged radiation exposure of the clinician and patient, e.g. due to a computer tomography/angiography, dependence on clinician's skill for placement and alignment and is subject to precision limitations, and prolonged and/or error-prone procedures, among others.

In addition, a high accuracy determination of the access site of the prior art method mostly requires costly computer tomography imaging, complex equipment/controls, or specific skills of a clinician, so that the prior art lacks a simple, easy to operate, and cost-effective method and system for determination of an access site.

An automated determination of an access site might give rise to a fully automated catheterization, as the position of the access site can be electronically passed directly to a connected system/the control unit for insertion of a catheter/needle/guidewire without necessarily requiring a human-machine interface for carrying out vascular access procedure.

WO 2021/161516 A1 discloses a 6-axis robot for aligning an ultrasound probe such that a cross-sectional center of a blood vessel is arranged in an echo image or rotated such that a longitudinal cross-section of the blood vessel is aligned in the echo image.

US 2003/167005 A1 discloses a probing method and system on a robot arm with two rotational and two translational degrees of freedom which allow for determining a center line of a blood vessel and an image plane to be positioned parallel to the center line of the blood vessel.

In particular, the prior art lacks to provide a reliable method for customized access site determination based on a patient's anatomy and corresponding automated control of movement and orientation of an imaging probe, and in particular corresponding pre-alignment of medical device.

The prior art lacks a computer implemented method that determines, in particular in a fully automated manner, an access site, in particular a transfemoral access site. In addition, the prior art lacks optimized and precise computer implemented positioning and alignment of an imaging probe for determining the access site, taking into account a patient's anatomy and a trajectory and an orientation of the vascular elongated bodily structure to be accessed.

It is the object of the present invention to overcome these and other disadvantages of the prior art, and provide a computer implemented method, a robotic system, and a computer program product for determining an access site for intravascular access. These objects are solved by a computer implemented method, a robotic system, and a computer program product as defined in the independent claims. Further embodiments result from the dependent claims.

A computer implemented method for determining an access site for an intravascular access for a diagnostic or a surgical procedure, preferably executed on at least one control unit, according to the invention comprises the following steps.

The method comprises (a) receiving two- or three-dimensional image data of an elongated bodily structure of a patient transmitted from an imaging probe, in particular an ultrasound probe.

The method comprises (b) positioning the imaging probe by actuation of a positioning module in a first orientation based on the image data such that a two-dimensional imaging plane is aligned essentially orthogonally to a longitudinal axis of the elongated bodily structure.

The method comprises (c) positioning the imaging probe by actuation of the positioning module based on the image data in the first orientation along the longitudinal axis of the elongated bodily structure, in particular in a proximal direction of the elongated bodily structure.

The method comprises (d) analysing the received two- or three-dimensional image data in view of the presence of a landmark of the elongated bodily structure, in particular a bifurcation of the elongated bodily structure.

The method comprises (e) repeating the previously described steps (a) - (c) if no landmark is detected.

If a landmark is detected based on the received image data in a step (e) the imaging probe is positioned by actuation of the positioning module based on the image data from the first orientation to a second orientation, such that the longitudinal axis of the elongated bodily structure is essentially aligned with the two-dimensional imaging plane in proximity of the landmark, in particular at the landmark.

The method comprises (g) determining the access site based on the image data, in particular the image data in the second orientation.

Within the present application, the proximal direction is defined as directed towards the patient's heart and the distal direction is defined as directed away from a patient's heart.

In a preferred embodiment, the image data is only two-dimensional. Alternatively, the image data may be three-dimensional image data, in particular time resolved Doppler three-dimensional data, e.g. indicative of the blood flow within the elongated bodily structure.

The method may comprise positioning the imaging probe by actuation of the positioning module based on the image data in the first orientation along the longitudinal axis of the elongated bodily structure distally, and optionally proximally, from the landmark, in particular at least 1 cm, 2, cm 3 cm, or 5 cm proximally and distally from the landmark.

The vascular elongated bodily structure may alternatively be a different artery than the femoral artery or a vein, in particular the carotid artery which may also be used for catheterization during surgical procedures.

The landmark may be a bifurcation of the elongated bodily structure, in particular the lateral circumflex femoral artery or the bifurcation of the profunda femoris artery (deep femoral artery). The landmark may also be formed by the femoral head detected by the imaging probe.

Alternatively or additionally, the landmark may be detected based on the image data combined with additional data, such as pre-operative data, in particular computer tomography data. The pre-operative data may be raw pre-operative data or processed pre-operative data, in particular segmented pre-operative data. The pre-operative data may include characteristics, such as vascular plaque amount and/or location within a vascular elongated bodily structure which may be otherwise difficult to detect when e.g. using an ultrasound imaging probe. This may be particularly useful for detecting a landmark formed by a region which has more/fewer vascular plaque, in particular calcifications, such that the access site can be determined while minimizing a risk of plaque detachment, e.g. by avoiding calcifications, in a subsequent surgical step. The method or a pre-operative method may comprise the step of detecting the characteristics in the pre-operative data, in particular within a region of interest corresponding to a vicinity of, at least, one landmark in the pre-operative data. This detection can be carried out by using a threshold algorithm or a neural network, in particular by using the control unit.

This allows to determine a more accurate trajectory of the elongated bodily structure and thus determination of an access site.

The method or a pre-operative method may comprise the step of determining the access site in the pre-operative data based on the detected characteristic and the landmark(s) in the pre-operative data.

The method may comprise the step of registration of the two- or three-dimensional image data with the pre-operative data. Prior to the method or when carrying out the method, the access site, the characteristic (e.g. calcification), and/or the landmarks may be defined in the pre-operative data. The access site may be defined by a longitudinal position and in particular a circumferential position for optimizing path-planning of a posterior surgical intervention. This may allow to precisely locate the longitudinal/circumferential position of the access site, the characteristic(s), and/or the landmark of the pre-operative model.

This registration of the image data and the pre-operative data allows to determine the access site / characteristic(s), and/or landmark in a more precise and reliable manner which reduces inaccuracies and errors that may result in clinical complications. Such accuracy can be obtained thanks to an efficient planning using a high-resolution pre-operative data, and also in particular, based on a precise robotic localization when acquiring the two-or three-dimensional image data.

The method may comprise successively repeating / adjusting the step of registration of the two- or three-dimensional image data and the pre-operative data, based on newly received image data.

The method step of the registration may comprise detecting pose / trajectory data of the robotic system, e.g. a robotic arm, in particular with respect to the solid support of the robotic system, indicative of the spatial position of the imaging probe to enable a more precise registration of the image data and the pre-operative data.

The step of registration may comprise aligning an orientation of the two- or three-dimensional image data with the pre-operative data in particular by carrying out an iterative closest point (ICP) algorithm.

The method may comprise a step of computing an orientation / trajectory of the elongated bodily structure in a virtual representation of the elongated bodily structure in the two- or three-dimensional image data with respect to a virtual representation of the elongated bodily structure within the pre-operative data. This enables a precise registration irrespective of the orientation or position of the patient.

The method may further comprise the step of merging the virtual representations of the elongated bodily structure of the two- or three-dimensional image data and the pre-operative data and in particular display the merged model on a display. This may e.g. be achieved by using a three-dimensional centerline detection algorithm and/or a landmark detection algorithm (e.g. femoral head or femoral bifurcation).

Merging the data may include transformations such as a scale adjustment, a size normalization, and/or a spatial orientation alignment.

The method may further comprise conversion of a spatial position and/or circumferential data, in particular of the access site, by the control unit into movement commands of the positioning module. This allows to transmit the movement commands to reliably move the positioning module to the determined access site for a subsequent surgical intervention.

The imaging probe(s) may be formed by an ultrasound probe or a fluoroscopic probe. The ultrasound probe may be a linear ultrasound probe or a convex ultrasound probe.

The method may comprise a step for identification of a specific feature. This can be carried out by an image segmentation of the two-dimensional image data via the control unit which is adapted to receive the image data. The step may comprise cropping the two-dimensional image data and/or applying at least one filter or mathematical operation, such as a gaussian filter, anisotropic diffusion filter, guided filter, median filter, low pass, and high pass filter to the image data.

Alternatively or additionally, the method may comprise a feature identification step which comprises applying a machine-learning algorithm to the image data, in particular a convolutional neural network. This convolutional neural network can further be used to determine a cross-sectional contour of the elongated bodily structure. The convolutional neural network may be trained by using a training data set of cross-sectional two-dimensional image data of elongated bodily structures.

The method may comprise positioning the imaging probe in the first orientation based on the image data by actuation of the positioning module, prior to positioning the imaging probe in the second orientation, such that the elongated bodily structure is centrally arranged in a field of view of the imaging probe.

Alternatively or additionally, the method comprises repositioning the imaging probe in the first orientation by actuation of the positioning module prior to moving the imaging probe to the second orientation, such that the longitudinal axis of the elongated bodily structure coincides with a rotational axis of the imaging probe by the positioning module.

This may allow for a simpler processing of the image data in the first orientation and/or easier conversion of the imaging probe to the second orientation, e.g. only by rotating the imaging probe in a single degree of freedom.

The step of positioning the imaging probe in the second orientation may comprise rotating the imaging probe with respect to the elongated bodily structure. The rotation may initially be around a fixed angle of between 80° and 100°, in particular between 80° and 95°.

This allows the same imaging probe to be used in the first and second orientation for the determination of the access site. This rotation allows the imaging probe to be positioned approximately in the second orientation, or accurately positioned based on a subsequent small-step scan after the rotation.

In addition, positioning in the second orientation allows monitoring a catheterization in a two-dimensional imaging plane in the second orientation once a landmark was detected.

The step of positioning the imaging probe in the second orientation may comprise rotating the imaging probe with respect to the elongated bodily structure. An angular position of the two-dimensional imaging plane with respect to the elongated bodily structure can be determined in parallel.

Determining the angular position may be based on the image data detected while rotating, in particular based on the increasing cross-section of the detected elongated bodily structure within the image data while rotating.

Alternatively or additionally, determining the angular position during rotation may be based on detecting a characteristic of at least one region of interest of the image data, in particular at least one region of interest arranged at one outer edge of the two-dimensional image data. The angular position at which the second orientation is reached can be defined based on reaching an extremum, in particular a local extremum, of the characteristic.

The extremum may be a maximum or minimum, in particular a local maximum or minimum.

This allows for an optimized positioning of the imaging probe in the second orientation even for tortuous elongated bodily structures, in particular in a fully automated manner.

The characteristic may be based on a detected intensity distribution in the region of interest. The characteristic may be determined via a mathematical operation carried out using the intensity distribution in the region of interest, in particular the mean, median, maximum, minimum, a numeric integration of a fit function, or other suitable mathematical operations.

Another aspect of the invention relates to a computer implemented method for determining an access site for intravascular access for a surgical intervention, preferably executed on at least one control unit.

The method comprises (a) receiving first two-dimensional image data of an elongated bodily structure of a patient transmitted from a first imaging probe, in particular a first ultrasound probe.

The method comprises (b) positioning the first imaging probe by actuation of a positioning module such that a first two-dimensional imaging plane of the first imaging probe is aligned essentially orthogonally to a longitudinal axis of the elongated bodily structure.

The method comprises (c) positioning the first imaging probe by actuation of the positioning module based on the first image data along the longitudinal axis of the elongated bodily structure, in particular in a proximal direction of the elongated bodily structure. During this movement, the first imaging plane preferably positioned orthogonally to the longitudinal axis as described in step (b).

The method comprises (d) analysing the received first two-dimensional data in view of the presence of a landmark of the elongated bodily structure, in particular a bifurcation of the elongated bodily structure.

The method comprises repeating the steps (a) - (d) if no landmark is detected.

If a landmark is detected based on the received first image data two-dimensional image data of a second imaging probe are received.

The second imaging probe has a second two-dimensional imaging plane which is arranged at an angle with respect to the first two-dimensional imaging plane.

The second two-dimensional image data is received while the longitudinal axis of the elongated bodily structure is essentially aligned with the second two-dimensional imaging plane in proximity of the landmark, in particular at the landmark.

The method comprises the step of determining the access site, in particular based on the second image data.

The second two-dimensional imaging plane may have an angle with respect to the first two-dimensional imaging plane, in particular an angle between 80° - 100°, preferably between 80° and 95°, and especially between 85° and 90°.

The method may comprise the step of positioning a contact surface of the imaging probe which is mounted on the positioning module on the skin or mucosa or endothelium or eye cornea of the patient to image the elongated bodily structure.

The method may optionally comprise positioning the second imaging probe by actuation of the positioning module based on the first and/or second image data.

The step of positioning the contact surface may be based on guidance data detected by a guidance sensor, in particular an optical guidance sensor, a capacitive guidance sensor, or an inductive guidance sensor, which is connected to the at least one control unit. Alternatively or additionally, the step of positioning the contact surface may be based on a predetermined scanning pattern, in particular a geometric scanning pattern.

This allows a reliable placement of the contact surface even prior to contacting the patient, so that, for example, prior to an ultrasound imaging probe being functionally operatable, the contact surface can be guided based on the guidance data. The imaging probe may alternatively be adapted to function as the guidance sensor by being configured for measuring a distance to the patient, e.g. by having a ranging ultrasound probe which carries out a time-of-flight measurement of ultrasound pulses.

Once the skin or mucosa or endothelium or eye cornea of the patient is contacted by the contact surface, the field of view of the imaging probe, an imaging depth of the imaging probe, the spatial position of the imaging probe, and/or the force applied by the contact surface to the skin or mucosa or endothelium or eye cornea of the patient may be adjusted based on the image data.

The control unit and the positioning module can be adapted for guiding the contact surface based on the guidance data or the predetermined scanning pattern, in particular together with the image data of the imaging probe. The guiding is preferably carried out during positioning of the imaging probe in the first orientation.

The method may comprise the step of receiving force data from a force sensor of the positioning module indicative of an engagement pressure of the contact surface of the imaging probe with the skin or mucosa or endothelium or eye cornea of the patient. The method may further comprise the step of positioning the contact surface of the imaging probe on the skin or mucosa or endothelium or eye cornea of the patient for imaging the elongated bodily structure at a specific force value, in particular selected from force value range, by actuation of the positioning module based on the force data and preferably the image data.

This allows to ensure that the contact surface contacts the patient's skin or mucosa or endothelium or eye cornea with sufficient force to enhance the imaging quality of the elongated bodily structure by the imaging probe.

The force sensor may be a force torque sensor, in particular a six-axis force torque sensor which is adapted to measure three forces and three torques.

The force sensor can be connected to the at least one control unit and the positioning module may be controlled via the control unit based on a force control loop to contact the patient's skin or mucosa or endothelium or eye cornea with the specific force value or with a specific force value within a specific force value range.

The method may comprise the step of determining if the elongated bodily structure in the two- or three-dimensional image data is an artery, in particular the femoral artery, or a vein, in particular the femoral vein. This determination may comprise applying different engagement pressures, in particular gradually increasing / decreasing the engagement pressures, and detecting a deformation of a shape of the elongated bodily structure in the two- or three-dimensional image data based on the different engagement pressures. This allows to determine if the elongated bodily structure is an artery or a vein, since arteries are deformable to a lesser degree than veins.

In addition or alternative, the method step of registration of the two- or three-dimensional image data and the pre-operative data may comprise applying different engagement pressures, in particular gradually increasing / decreasing the engagement pressures of the contacting surface, and detecting the deformation of the shape of the elongated bodily structure. Based on the detected deformation due to the different engagement pressures, the registration accuracy may be increased.

The control unit and the positioning module may be adapted for positioning the contact surface against the skin or mucosa or endothelium or eye cornea with a different specific force value when imaging and when moving the imaging probe by actuation of the positioning module, in particular at a lower force when moving the imaging probe than when imaging via the imaging probe.

The guidance data, the force data, and/or the image data indicative of incorrect positioning of the contact surface of the imaging probe with respect to the elongated bodily structure may be used to reposition the contact surface of the imaging probe by actuation of the positioning module.

This allows repositioning the contact surface if e.g. the patient moves with respect to the contact surface or a clinician/patient moves the contact surface, for example by accidentally touching the positioning module.

The control unit can be adapted to determine an area of contact of the contact surface of the imaging probe and the patient's skin or mucosa or endothelium or eye cornea based on the force data, the image data, and optionally the guidance data. The control unit may control the positioning module to maximize the area of contact with the patient's skin or mucosa or endothelium or eye cornea based on this data.

The force sensor may thus allow positioning of the contact surface with respect to a spatial position/orientation of the patient's skin or mucosa or endothelium or eye cornea such that the area of contact is maximized and the image quality enhanced.

In a preferred embodiment, repositioning the contact surface is based on all the received guidance data, the force data, and the image data.

The two-dimensional section of the elongated bodily structure, and in particular the landmark, may be anatomically identified by the control unit based on the image data in real time based on at least one algorithm selected from (i) and image segmentation algorithm and (ii) a machine-learning algorithm, in particular based on a convolutional neural network. The algorithm may calculate a confidence map, in particular additionally based on a scan line integration or a random walk algorithm.

This real time identification of a two-dimensional section of the elongated bodily structure, and in particular the landmark, allows the method to be carried out quickly and preferably in a fully automated manner.

The confidence map can be used to optimize image quality by considering the measurement errors of the two-dimensional image data depending on the direction of incidence, angular position, and transmission during image data acquisition. The confidence map may be derived using a general confidence control or a target specific confidence control.

The method may comprise the step of carrying out a three-dimensional reconstruction of the elongated bodily structure in real time based on a plurality of the detected two-dimensional image data.

Carrying out a three-dimensional reconstruction of the elongated bodily structure may include registration of the respective two-dimensional image data corresponding to sections of the elongated bodily structure, in particular image data acquired in the first orientation and/or second orientation. The virtual three-dimensional reconstruction may also include a three-dimensional virtual reconstruction of the landmark.

This allows to consider the longitudinal course of the elongated bodily structure when determining the access site. The spatial position of the access site may be determined based on the three-dimensional reconstruction and/or the two-dimensional image data in the second orientation with an increased precision.

The control unit may further carry out an augmentation of the three-dimensional reconstruction by including additional data, such as computer tomography data/fluoroscopy data, in particular additional data indicative of vascular plaque.

The positioning module preferably has a mounting section for the imaging probe. The method may comprise a step of moving the imaging probe along six degrees of freedom by actuation of the positioning module and hence the mounting section.

The positioning module may be adapted for moving and orienting the imaging probe, and in particular the contact surface, operated by the control unit in six degrees of freedom.

The positioning module may be formed by a robotic arm which is arranged in proximity of a terminal end of the positioning module. The positioning module may be adapted for moving the imaging probe in a range of at least 20 cm, in particular at least 30 cm, preferably at least 40 cm, along the elongated bodily structure of the patient.

This allows for a sufficient range of motion for movement along the longitudinal axis of the elongated bodily structure, preferably in a fully automated manner.

The access site may be determined, such that a medical device for insertion into the elongated bodily structure is insertable through skin or mucosa or endothelium or eye cornea at the access site to enter the elongated bodily structure, in particular a lumen of the elongated bodily structure. The access site may be determined in a range between 1 cm and 6 cm proximally from the landmark, in particular in a range between 1.5 cm and 4 cm proximally from the landmark.

This allows for a subsequent safe insertion of a medical device after carrying out the previously described method without being affected by the landmark.

The medical device, in particular a catheter, puncturing element, or guidewire, may be further mechanically connected to the imaging probe. In particular, it may be movably connected to the imaging probe, preferably in two, three, four, five, or six degrees of freedom.

The method may further comprise positioning a medical device parallel to the two-dimensional image plane in the second orientation. Alternatively, the medical device may be pre-aligned with the two-dimensional imaging plane. This allows that the medical device, e.g. a catheter, may be oriented to be insertable into the access site in a direction within the imaging plane of the imaging probe in the second orientation.

The method may comprise the step of displaying the two-dimensional image data and/or three-dimensional reconstruction of the elongated bodily structure on a display device in real time.

This allows precise monitoring of the computer implemented method by a clinician.

Another aspect of the invention relates to a robotic system which comprises a control unit, a positioning module, and in particular an imaging probe mountable on the positioning module. The robotic system has means adapted for executing the steps of the computer implemented method previously described. The imaging probe may be an ultrasound probe, in particular a linear or convex ultrasound probe.

Another aspect of the invention relates to a computer program product which comprises instructions that cause the previously described robotic system to perform the previously described computer implemented method steps.

Further embodiments of the invention and improvements of the described embodiments will become apparent with the following nonlimiting description of embodiments.

Figure 1A: a perspective view of an imaging probe formed by an ultrasound probe or fluoroscopic probe which is movable and positionable by a positioning module in six degrees of freedom,

Figures 1B and 1C: a schematic view of the imaging probe of Fig. 1A arranged in a first orientation and a second orientation and the corresponding image data,

Figure 2: a perspective view of a first embodiment of a robotic system for carrying out the computer implemented method having a control unit, a positioning module, and an imaging probe,

Figure 3: a perspective view of a second embodiment of a robotic system for carrying out the computer implemented method having a control unit, a positioning module and a first and second imaging probe,

Figures 4A and 4B: exemplary two-dimensional image data of an elongated bodily structure and a landmark detected in the first orientation of an imaging probe at different longitudinal positions of the elongated bodily structure,

Figures 4C - 4E: a schematic method of pre-processing the two-dimensional image data acquired by the imaging probe,

Figures 5A - 5E: image data acquired during positioning the imaging probe of Fig. 2 from the first orientation to the second orientation and beyond the second orientation,

Figure 5F: exemplary steps of the computer implemented method for positioning the imaging probe of Fig. 2 from the first orientation to the second orientation,

Figures 6A and 6B: a schematic representation of determining an access site based on image data acquired by the probe in the second orientation and/or on the three-dimensional reconstruction of the elongated bodily structure,

Figures 7A and 7B: exemplary two-dimensional image data acquired by the probe in the first orientation with the elongated bodily structure not centrally arranged and centrally arranged within the image and a corresponding confidence map respectively,

Figure 8: a graph of a target position and a current position of the center of a femoral artery in the two-dimensional image data in pixel coordinates over time,

Figures 9A - 9C: a schematic representation of a plurality of image data acquired during movement of the imaging probe along the longitudinal direction of the elongated bodily structure to carry out a three-dimensional reconstruction,

Figure 10: exemplary segmented pre-operative computer tomography data including locations of calcifications on which a spatial position of a landmark and an access site was defined,

Figures 11A and 11B: the two-dimensional image data acquired in the first orientation which are successively augmented to form a virtual model of the cross-sectional contour of the elongated bodily structure and a landmark formed by a bifurcation,

Figure 11C: a schematic representation of a plurality of the cross-sectional contours of an elongated bodily structure which are to be registered to pre-operative data.

Figure 1A shows a perspective view of an imaging probe 5 formed by an ultrasound probe which is movable and positionable by a positioning module 6 in six degrees of freedom (see Figs. 2 and 3). The three degrees of freedom depicted by double-headed arrows on the left side of Fig. 1A denote an out-of-plane movement with respect to a two-dimensional imaging plane 53 of the imaging probe 5. The three degrees of freedom depicted as double-headed arrows on the right side of Fig. 1A denote an in-plane movement which does not affect the orientation of the two-dimensional imaging plane 53. Two-dimensional images 3, 30 (see Figs. 1B and 1C) which are detected via the imaging probe 5 are transmitted from the imaging probe 5 to a control unit 2 which is adapted to control the positioning module 6 (see Figs. 2 and 3). A contact surface 55 of the imaging probe 5 in Fig. 1A is arranged adjacent to the skin of a patient for imaging an elongated bodily structure 4.

The elongated bodily structure 4 is a vascular elongated bodily structure, preferably the femoral artery of a patient.

The figures 1B and 1C show a schematic view of the imaging probe 5 of Fig. 1A arranged in a first orientation 51 and a second orientation 52 and the corresponding images 3, 30 detected in the first and second orientation 51, 52 respectively.

Figure 1B shows that the imaging probe 5 is arranged in the first orientation 51 in which the two-dimensional imaging plane 53 is arranged perpendicular to a longitudinal axis L of the elongated bodily structure 4. Two-dimensional images 3 acquired when moving the imaging probe 5 in the first orientation 51 along the longitudinal axis L during the computer implemented method are transmitted to a control unit 2 (see Figs. 2 and 3). In the first orientation 51, a landmark such as a bifurcation of the elongated bodily structure 4, e.g. of the femoral artery forming the profunda femoral artery may be detected during the computer implemented method (see Figs. 4A and 4B).

Figure 1B further shows that the control unit 2 and the positioning module 6 (see Fig. 2) are further adapted for positioning the imaging probe 5 in the first orientation 51, such that the longitudinal axis L of the elongated bodily structure 4 is centrally arranged within the two-dimensional image 3 of the imaging probe 5. The imaging probe 5 is arranged in such a way that the contact surface 55 (see Fig. 1A) is applied to the patient's skin 10 over as large a surface area as possible and at a specific pressure value. Centrally arranging a cross-sectional contour of the elongated bodily structure 4 may be carried out based on feature extraction via image segmentation and/or based on machine-learning. Centrally arranging the cross-sectional contour in an automated manner further reduces the computational demand required for the feature extraction and actuation control via the positioning module.

Image feature extraction by segmentation is computationally less demanding and may have fewer false positives but is more error-prone due to artifacts in the images 3, 30.

Feature extraction by a machine-learning algorithm is computationally more demanding and may produce more false positives but is less dependent on the parameters of the imaging probe 5, such as an ultrasound probe or fluoroscopic probe, and less error-prone due to artifacts in the images 3, 30.

A cross-sectional contour of the elongated bodily structure 4 may be positioned such that a geometric center of mass of the contour coincides with the rotational axis of the imaging probe 5. This allows the imaging probe 5 to be moved from the first orientation 51 to the second orientation 52 by merely rotating the imaging probe 5 in a single degree of freedom.

Consequently, the imaging probe in the first orientation 51 is arranged such that the longitudinal axis L of the elongated bodily structure 4 intersects an axis of rotation of the imaging probe 5.

In the second orientation 52, the two-dimensional imaging plane 53 contains the longitudinal axis L of the elongated bodily structure 4. The second orientation 52 allows for precise determination of an access site through the skin 10 of a patient and also monitoring of medical devices in a subsequent surgical catheterization step.

The control unit 2 (see Figs. 2 and 3) is adapted for moving the imaging probe from the first orientation 51 to the second orientation 52 when a landmark 7 (see e.g. Figs. 4A and 4B) is detected while moving along the longitudinal axis of the elongated bodily structure 4.

The imaging probe 5 is movable between the first and second orientation 51, 52 in a fully automated manner by a positioning module 6 controlled via the control unit 2 (see Figs. 2 and 3).

Figure 2 shows a perspective view of a first embodiment of a robotic system 101 for carrying out the computer implemented method having a control unit 2, a positioning module 6, and an imaging probe 5. The imaging probe 5 is connected at a mounting section 13 of the positioning module 6 formed by a robotic arm such that the imaging probe 5 is movable in six degrees of freedom for positioning a two-dimensional imaging plane 53 of the imaging probe 5. The degrees of freedom for positioning of the imaging probe 5, e.g. on the skin of a patient, or moving the imaging probe 5 along a longitudinal axis of the elongated bodily structure, may be constrained to fewer, in particular less than five, four, or three degrees of freedom such that the computational demand can be reduced.

Figure 2 shows that the imaging probe 5 and the positioning module 6 are connected to the control unit 2 for data transmission, such that the positioning module 6 is operable based on instructions provided by the control unit 2 taking into consideration the detected images. Figure 2 shows that the imaging probe 5 is arranged at the mounting section 13 in the previously described first orientation 51 (see Figs. 1A and 1B).

The control unit 2 is further connected to a display 11 for displaying the two-dimensional images 3, 30 (see Figs. 1B and 1C) and/or a three-dimensional reconstruction of the elongated bodily structure and in particular the landmark (see Figs. 9A - 9C). This allows a clinician to monitor the complete determination of the access site in a computer implemented method.

The imaging probe 5 may be adapted to change the scanning depth, e.g. in a range of 20 mm - 100 mm, in particular in a range of 40 mm - 80 mm. The imaging probe 5 and control unit 2 may further be adapted for generating a Doppler ultrasound image 30 to improve the image data quality.

The images 3, 30 (see Figs. 1B and 1C) and in particular the three-dimensional reconstruction may be registered to preoperational data such as preoperational computer tomography data. This preoperational data may further improve the determination of an access site by, for example, registration of spatial data of vascular plaque to specific regions in the three-dimensional reconstruction. Otherwise, vascular plaque is rather difficult to detect using an ultrasound imaging probe as imaging probe 5.

The control unit 2 is preferably connected to a guidance sensor 8 (see Fig. 2). The guidance sensor 8 is formed by a camera, so that the control unit 2 is adapted to position the imaging probe 5 on the patient's skin based on guidance data. For example, the movement of the imaging probe 5 via the positioning module 6 can be controlled via a feedback loop based on the guidance data detected by the guidance sensor 8. Alternatively, the guidance sensor 8 may be formed by a near infrared imaging probe, a capacitive sensor, an ultrasound ranging probe or an inductive sensor which may be attached directly to the imaging probe 5 or at a terminal tip of the positioning module 6.

The control unit 2 is further connected to a force sensor 9 which is preferably integrated within the imaging probe 5. The force sensor 9 in Fig. 2 is formed by a six-axis force torque sensor which is adapted for detecting force and torque acting on the contact surface 55. The force sensor 9 provides the control unit 2 with force data when moving the contact surface 55 along the elongated bodily structure 4 and/or when positioning the contact surface 55 with the skin in the first and second orientation 51, 52 (see Figs. 1B and 1C). The control unit 2 is further adapted for controlling the positioning module 6 in such a manner that the contact surface 55 is positioned as congruently as possible to the skin based on the force data, the images, and in particular the guidance data. The control unit 2 is further configured to adjust a contact pressure of the contact surface 55 contacting the skin for optimization of an imaging quality of the imaging probe 5. Based on this data, the control unit 2 can adjust the contact pressure of the contact surface 55 to different contours of a skin surface and deformability of the patient's tissue.

This may be achieved by controlling the positioning module 6 via the control unit 2 in such a manner that a longitudinal direction of the imaging probe 5 is essentially arranged vertically with respect to a surface of the skin. The contacting pressure is preferably adjusted to a specific value, preferably selected from a range, via a feedback loop based on the force data, in particular when moving the imaging probe 5 in the first orientation 51.

The contact pressure of the contact surface 55 exerted on the skin of a patient may be restricted to remain below a threshold, e.g. below 40N, 50N, 60N, 70N, 80N, 90N, or 100N, to guarantee the safety of the patient.

After or during positioning of the imaging probe 5 on the patient's skin, the patient is scanned by moving the contact surface 55 of the imaging probe 5, e.g. in a predetermined or geometric scanning pattern, to determine the location of the elongated bodily structure 4. The system 101 is adapted to align the imaging probe 5 with the elongated bodily structure 4 by a raster, spiral, sinusoidal geometric scanning pattern or by a random walk positioning. In addition, the contacting force and/or the scanning depth may be adjusted during positioning based on the images and/or pressure data.

Subsequently, the imaging probe 5 is positioned in the first orientation 51 and moved along a longitudinal axis L of the elongated bodily structure 4 via the positioning module 6 until a landmark 7 is detected (see Figs. 4A and 4B).

After detection of the landmark 7 the imaging probe 5 is moved to a second orientation 52 (see Fig. 1C) such that a longitudinal axis of the elongated bodily structure 4 is aligned parallel to the two-dimensional imaging plane 53. Prior to moving the imaging probe 5 to the second orientation 52, the imaging probe may be advanced further along the elongated bodily structure 4 to collect additional images on the bodily structure 4 posterior the detected landmark 7.

The parallel positioning in the second orientation 52 may be achieved at precision by carrying out a sweeping scan in which the imaging probe 5, and thus imaging plane 53, is rotated from the first orientation 51 with a rotation axis of the imaging probe 5 running through a center of the elongated bodily structure 4. The sweeping scan preferably includes a rotation of more than 90° such that the imaging plane 53 is rotated beyond the configuration parallel to the longitudinal axis of the elongated bodily structure 4. In a preferred embodiment the sweeping scan is carried out by rotating the imaging probe 5 by an angle of less than 90°, in particular in a range of 60° - 90°, preferably 70° - 80°, especially 85° - 90°, and subsequently, carrying a finer scan in smaller angular steps beyond alignment parallel to the longitudinal axis of the elongated bodily structure 4.

The images 3 of this scan can be evaluated based on a characteristic of a feature within the image. This characteristic can, for example, relate to a summed-up intensity in a region of interest 12 of the images 3 (see Figs. 5A - 5F). Based on this characteristic of the images 3, the two-dimensional imaging plane 53 can be precisely positioned parallel to the elongated bodily structure 4 e.g. by determining the minimum intensity within the region of interest 12. Based on the images in this second orientation 52 the access site in a proximal direction from the landmark 7 can be determined and e.g. a subsequent vascular catheterization can be monitored.

Moreover, a translation scan may be performed in which the imaging probe 5 (when it is in the second orientation) is translated by translational movement of the imaging probe 5 perpendicular to the longitudinal direction of the elongated bodily structure 4 for further improving the determination of the access site.

The data acquired by the sweeping scan the translation scan and/or the images 3 are used by the control unit 2 to carry out a three-dimensional reconstruction of the elongated bodily structure 4 and the landmark 7 (see Figs. 4A and 4B).

After the imaging probe 5 was moved to the second orientation an access site 1 can be determined based on the images and in particular the three-dimensional reconstruction (see Figs. 6A and 6B) .

The imaging probe 5, the positioning module 6, the control unit 2, and in particular the guidance sensor 8 are adapted for carrying out the previous steps of the computer implemented method, in particular in a fully automated manner.

Figure 3 shows a perspective view of a second embodiment of a robotic system 101 for carrying out the computer implemented method for determining an access site having a control unit 2, a positioning module 6, and a first and second imaging probe 5, 50. The second embodiment of the robotic system 101 is different from the embodiment in Fig. 3 only by having a second imaging probe 50. The first and second imaging probe 5, 50 are arranged such that the first and second two-dimensional imaging planes 53, 503 are arranged at an angle of 90° with respect to each other.

This allows the imaging probes 5, 50 to detect images 3, 30 (see Figs. 1B and 1C) in a first and second two-dimensional imaging planes 53, 503 with minimized adjustment of the imaging probes 5, 50, in particular simultaneously. In addition, this arrangement of imaging probes 5, 50 allows for less movement by the positioning module 6 for arranging the first imaging probe 5 analogously to the previously described first orientation and the second imaging probe 50 analogously to the previously described second orientation.

The first and second images 3, 30 may be used by the control unit 2 for optimizing the positioning of the imaging probe 5 such that the imaging plane 53 is aligned orthogonally to a longitudinal axis L of the elongated bodily structure 4. The first and second images 3, 30 are further used to optimize an orientation of the second imaging probe 50 such that the longitudinal axis L of the elongated bodily structure 4 is aligned with the second imaging plane 503 in proximity of the landmark 7 (see Figs. 4A and 4B).

A previously described sweeping scan (see Fig. 2) can therefore be carried from a starting position in which the first imaging probe 5 has the first imaging plane 53 arranged orthogonally to the longitudinal axis L and the second imaging probe 50 is aligned/almost aligned such that the longitudinal axis L is within the second imaging plane 503. A finer scan by moving the second imaging probe 50 in smaller angular steps may be used to determine an optimized degree of rotation once a landmark was detected.

During moving the imaging probes 5, 50 along a longitudinal axis of the elongated bodily structure 4, during carrying out the sweeping scan, and optionally the translation scan, the first images 3 and the second images 30 are used by the control unit 2 to carry out a three-dimensional reconstruction of the elongated bodily structure 4 and the landmark 7 (see Figs. 4A and 4B).

After the second imaging probe 50 was positioned for parallel imaging of the bodily structure, an access site 1 can be determined based on the images and additionally or alternatively based on the three-dimensional reconstruction (see Figs. 6A and 6B) .

The imaging probes 5, 50, the positioning module 6, and the control unit 2 are adapted for carrying out the previously discussed steps of the computer implemented method, in particular in a fully automated manner.

Figures 4A and 4B show exemplary two-dimensional images 3 of an elongated bodily structure 4 and a landmark 7 detected in the first orientation of an imaging probe at different longitudinal positions of the elongated bodily structure 4. The elongated bodily structure 4 in Figs. 4A and 4B is the femoral artery and the landmark 7 is formed by a bifurcation of the femoral artery at the profunda femoral artery 41. The figure 4A shows images 3 of the imaging probe 5 in the first orientation 51 (see Figs. 2 and 3) at a location distally from the landmark 7. The figure 4B shows images 3 of the imaging probe 5 in the first orientation 51 in proximity of the landmark 7 detected once the imaging probe 5 was moved in a proximal direction towards the heart of the patient. Once the imaging probe 5 reaches the position of the landmark 7 formed by the bifurcation, the imaging probe 5, 50 (see Figs. 2 and 3) is converted to the second orientation. The landmark 7 may also be formed by a different anatomical structure, e.g. a section of the elongated bodily structure which is free from vascular plaque.

Figures 4C - 4E show pre-processing of the two-dimensional images 3 acquired by the imaging probe. The received two-dimensional images 3 (see Fig. 4C) may be cropped to the size of the image layer (see Fig. 4D). Alternatively, the image could be cropped to a region of interest 12, 121 (see Figs. 5A - 5E), in particular a region of interest adapted to the corresponding position/orientation of an elongated bodily structure 4 in the images 3. The pre-processing of the images 3 may further include filters and/or mathematical operations, such as image enhancement based on a confidence map, for enhancing the performance of the feature extraction, in particular by a machine-learning algorithm. Figure 4E shows that a cross-sectional contour 42 of the elongated bodily structure 4 was reconstructed in the two-dimensional images 3.

Figures 5A - 5E show two-dimensional images 3 acquired during moving the imaging probe 5 of Fig. 2 from a first orientation 51 to a second orientation 52 (see Figs. 1B and 1C).

Figure 5A shows images 3 from the imaging probe being arranged in the first orientation 51 in which a longitudinal axis is arranged essentially perpendicular to the two-dimensional imaging plane.

Figure 5B shows images 3 of the imaging probe which was rotated by approximately 40° with respect to the first orientation.

Figure 5C shows images 3 of the imaging probe which was completely converted to the second orientation so by approximately 90°.

Figures 5D - 5E show images 3 of the imaging probe being moved beyond 90° with respect to the first orientation and thus also beyond the parallel configuration of the second orientation. The figures 5A - 5E show regions of interest 12, 121 highlighted schematically by dashed rectangles. A first region of interest 12 may be arranged over a subsection of the image 3 or the entire image 3, in particular, so that the elongated bodily structure 4 is just inside the region of interest 12. Alternatively, a region of interest 121 may be arranged at an edge of the images 3, in particular on both lateral edges of the images 3. This allows that the elongated bodily structure 4 is only inside the region of interest 121 when the imaging probe is positioned in proximity of the second orientation.

Figure 5F shows exemplary steps of the computer implemented method for positioning the imaging probe 5 of Fig. 2 from the first orientation to the second orientation via actuation of the positioning module 6. The first step 102 of the procedure is acquisition of the images by the imaging probe.

In step 103 features, such as the intensity profile only in a region of interest 12, e.g. formed by an interpolated rectangle (see Figs. 5A - 5F), are extracted. The figure 5F shows that in particular the bottom right image coordinates br of an interpolated rectangle corresponding to the elongated bodily structure may be used in a further step 104 to determine the corresponding angular orientation $\theta_z^c$ of the imaging probe with respect to a first orientation in which the two-dimensional imaging plane is arranged perpendicular to a longitudinal axis of the elongated bodily structure (see Fig. 1B).

In general, the angular orientation $\theta_z^c$ may be determined based on the change in intensity of the pixels when rotating the imaging probe, in particular from a starting position in the first orientation.

The angular orientation $\theta_z^c$ can be determined precisely by detecting the increase of a contour of the elongated bodily structure in the images depending on the angular orientation $\theta_z^c$. A further step 105 of the exemplary method of Fig. 5F includes a query as to whether the angular orientation $\theta_z^c$ corresponds to a target angle of rotation $\theta_z^t$. If this is not the case, the control unit 2 and a positioning module 6 (see Figs. 2 and 3) further adjust the angular orientation by $\theta_z$ accordingly in step 106 based on the computed current position $\theta_z^c$, target position $\theta_z^t$. This process will be repeated in a closed feedback-loop until the target angular orientation $\theta_z^t$ is reached. The target angle of rotation $\theta_z^t$ may correspond to a rotation between 60° - 90°, in particular between 70° - 80°, to carry out a sweeping scan using a system 101 using one imaging probe 5 (see Fig. 2).

Alternatively, the rotation by a specific target angle $\theta_z^t$ of rotation may be carried out by a step motor.

If the target angular orientation $\theta_z^t$ was reached, in step 107 a sum of pixel intensities is computed in a first area prev_area within the image. This sum corresponds to an intensity. The first area may correspond to a specific region of interest 12, 121 (see Figs. 5A - 5F) or the entire image.

In a further step 108 in Fig. 5F, the angular position is increased continuously or incrementally, such as by an angle $+\Delta\theta_z$, e.g. by 1°, 0.1°, or 0.01°. In step 109 in Fig. 5F, the corresponding sum of pixel intensities of a second area current_area within the image corresponding to a total intensity is computed. In addition, it would be conceivable that this incremental movement is not made at constant steps, but rather approaches the target angular orientation with different incremental steps, e.g. as in a binary search or a coarse prior scan and refined subsequent scan.

Subsequently, a step 110 includes a query if the pixel counts in the second area current_area is higher than the pixel counts in the first area prev_area. If this is not the case, the second area will be defined as the first area prev_area in step 111 and the steps 108, 109, 110 including a rotation by $+\Delta\theta_z$, computing a new second area current_area, and the query will be repeated.

If the second area current_area was higher than the previous area, the angular position is corrected by moving the imaging probe reversely by an incremental angle $-\Delta\theta_z$ in step 112 and the imaging probe is put in the second orientation in which the two-dimensional imaging plane of the imaging probe coincides with a longitudinal axis of the elongated bodily structure.

The figures 6A and 6B show a schematic representation of determining an access site 1 to the elongated bodily structure 4 formed by a femoral artery based on images in the second orientation and/or three-dimensional reconstruction of the elongated bodily structure 4.

Figures 6A and 6B show a section of a robotic system 101 (see Figs. 2 and 3) comprising an imaging probe 5 formed by an ultrasound probe or fluoroscopic probe which is attached to a solid support 14. The solid support 14 which is movable by a positioning module of the robotic system 101 is attached to an introducer unit 15 which carries a puncturing element 16 formed by a cannula for insertion into the access site 1 of the elongated bodily structure 4. The figures 6A and 6B show an artery penetration angle 18 which may, e.g. be arranged in an angular range between 15° to 60°, in particular 30° to 45° with respect to a longitudinal axis L of the elongated bodily structure 4. Thus, the access site 1 to the elongated bodily structure 4 and the penetration angle 18 determine a spatial location of an access path 19 through the skin of a patient. These angular values are known to a person skilled in the art, such as for carrying out catheterization, e.g. via the Seldinger procedure.

Figure 6A shows that the introducer unit 15 is attached at the artery penetration angle 18 to the solid support 14.

Figure 6B shows that the introducer unit 15 is movably attached to the solid support 14 such that the penetration angle 18 can be adjusted (see Fig. 6B, double-headed dashed arrow). Additionally, Fig. 6B shows that the introducer unit 15 has a catheter 17 which may be functionally connected to the puncturing element 16 for catheterization of the elongated bodily structure 4.

The control unit may further be adapted to control the movement of the introducer unit 15, in particular orienting and positioning the puncturing element 16 and/or the catheter 17 in one or more degrees of freedom prior to carry out the surgical step of a surgical intervention.

Figures 7A and 7B show exemplary two-dimensional images acquired by the probe in the first orientation with the elongated bodily structure 4 not centrally arranged (Fig. 7B) and centrally arranged (Fig. 7A) within the image 3 and a corresponding confidence map 20 respectively. A control unit is adapted to determine and display a cross-sectional contour 42 of the elongated bodily structure 4 (see Figs. 7A and 7B, white contour).

The control unit is adapted to carry out an identification of the cross-sectional contour 42 and in particular of a landmark in the images 3 in real time based on the previously described feature extraction.

The confidence map 20 in Fig. 7A and 7B visualizes the quality of the two-dimensional image 3 with dark regions corresponding to low confidence/low quality regions and bright regions to high confidence/good quality regions in the image 3. The control unit of the robotic system carries out an optimization of the image 3 based on the confidence map 20 and controls the positioning module to optimize the positioning of the imaging probe, e.g. such that the cross-sectional contour of the elongated bodily structure 4 is arranged centrally in the image 3 or at a position corresponding to a rotational axis of the imaging probe.

The confidence map 20 in Figs. 7A and 7B further includes a white line 21 representing the position of the confidence weighted barycentre of the elongated bodily structure 4 and a black line 22 representing a centre of the cross-sectional contour 42. The confidence weighted barycentre corresponds to a weighted average of all the values of the confidence map. The control of the positioning module 6 (see Figs. 2 and 3) by the control unit 2 may be adapted based on a general confidence control and/or a target specific confidence control.

In the general confidence control, a general image quality is optimized and the weighted barycentre is aligned in the center of the image.

In the target specific confidence control, the imaging data is optimized in a subsection of the image, preferably around the contour of the elongated bodily structure 4.

Figure 8 shows a graph of a target position and a current position (u) of a center of a cross-sectional contour of the elongated bodily structure formed by the femoral artery in the two-dimensional images over time in seconds with an induced disturbance between T1 and T2. The figure 8 shows that the imaging probe was positioned in a first orientation (see Figs. 1B, 7A and 7B) prior to T1 such that the cross-sectional contour of the elongated bodily structure was arranged at the target position (Fig. 8, dashed line). The disturbance may relate e.g. to an accidental disturbance such as a patient moving with respect to the robotic system or accidentally pushing the imaging probe.

The figure 8 shows that the imaging probe is repositioned by the control unit and positioning module between T2 and T3 to the target position in the images such that the imaging probe is re-positioned at the target position if an incorrect positioning was detected.

The incorrect positioning may be detected by the control unit 2 based on the guidance data via the guidance sensor 8, the force data via the force sensor 9, the images 3, 30 via the imaging probe(s) 5, 50 and/or the three-dimensional reconstruction (see Figs. 2 and 3). The control unit 2 may carry out the re-positioning based on a machine learning algorithm or a control loop, e.g. a PID control loop.

This re-positioning may be carried out in the first and/or second orientation of the imaging probe. Even though Fig. 8 only shows the spatial coordinate u in pixels, e.g. relating to an x-coordinate or y-coordinate in the two-dimensional images, correcting the incorrect positioning may involve spatial re-positioning of the imaging probe by translation and/or rotation of the imaging probe, e.g. along six degrees of freedom of a positioning module of the robotic system. This may in particular be the case for correcting an incorrect positioning of the imaging probe by positioning the imaging probe in the first or second orientation as previously described. From a point in time T3, the imaging probe is re-positioned in the desired first or second orientation to a longitudinal axis of the elongated body structure.

The figures 9A to 9C show a schematic representation of a plurality of two-dimensional images 3 acquired during movement of the imaging probe in the first orientation along the longitudinal direction of an elongated bodily structure 4. The images 3 are used to carry out a three-dimensional reconstruction 23 of the elongated bodily structure 4 which is schematically shown in Fig. 9C. The three-dimensional reconstruction 23 can be carried out by the control unit 2 in real time based on a stack 30 of the two-dimensional images 3 and an identified cross-sectional contour 42 of the elongated bodily structure 4 (see e.g. Fig. 9C). The three-dimensional reconstruction 23 may be further enhanced/refined by including additional two-dimensional images acquired in the second orientation of the imaging probe, images of a sweeping or translation scan (see e.g. Figs. 5A - 5F). Moreover, the three-dimensional reconstruction 23 may be improved by including additional data, such as pre-/intraoperative computer tomography data or fluoroscopy data, indicating spatial positions of plaque, in particular vascular calcifications.

Figure 10 shows exemplary pre-operative segmented computer tomography data 24 including locations of calcifications 25 on which a spatial position of a landmark 7 formed by a bifurcation and an access site 1 was defined.

Raw pre-operative computer tomography data 26 are processed to obtain a segmentation of the elongated bodily structure, e.g. by using thresholding or a convolutional neural networks. The segmented pre-operative data 24 in Fig. 10 may include only a model of the elongated bodily structure, and in particular calcifications 25 and/or an access site 1.

The access site 1 is defined in the segmented pre-operative data 24, e.g. by using a control unit, at a longitudinal spatial position between 1 - 3 cm distally from the landmark 7. In addition, the access site 1 may be defined by a particular circumferential position which avoids the calcifications 25. Alternatively or additionally, different landmarks, in particular anatomical structures such as the femoral head may be used for increasing the registration accuracy and/or the access site determination.

The plurality of two-dimensional image data 3 which are registered to the segmented pre-operative data 24 may be used to optimize the access site 1 determination (see Fig. 11C). The control unit is configured to convert the optimal spatial orientation and position of the access site 1 based on the registration to motor coordinates of the positioning module and move the positioning module accordingly, such that a subsequent surgical procedure can be performed in a simple and reliable manner.

The figures 11A and 11B show the two-dimensional image data 3 (left side) acquired in the first orientation which are successively augmented to form a virtual reconstruction 23 (right side) of the cross-sectional contours of an elongated bodily structure 4 and a landmark 7 formed by a bifurcation. The control unit is adapted to derive the virtual reconstruction 23 based on the two-dimensional contours of the elongated bodily structure in each image and a spatial distance of the imaging probe in between the successively acquired two-dimensional image data 3 with respect to a solid support of a robotic system, e.g. a previously described robotic system. The spatial distance of the imaging probe along the elongated bodily structure can be determined based on encoders of a positioning module, e.g. of a previously described six degrees of freedom positioning module, such that a pose / trajectory of the positioning module can be taken into consideration (see Fig. 1A). In a preferred embodiment, the distance to the solid support comprising a stand of the robotic system defines a reference point from which a relative spatial distance can be determined without having to move the robotic system.

The figure 11C shows a schematic representation 23 (see Figs. 11A and 11B) of a plurality of cross-sectional contours of an elongated bodily structure which are to be registered to the segmented pre-operative three-dimensional computer tomography data 24. Based on the registration of the two-dimensional image data, and in particular the representation 23 comprising the contours of the elongated bodily structure, with the segmented pre-operative data 24, the precision of detecting a landmark 7, e.g. formed by a bifurcation, may be increased. Furthermore, the segmented pre-operative computer tomography data 24 allows for determining characteristics, such as vascular plaque, in particular calcifications, which are not easily visible in ultrasound image data. By merging the two- or three-dimensional data with the segmented pre-operative data 24, a spatial orientation and a position of the characteristics and/or the landmark can be accurately reconstructed, e.g. to avoid vascular plaque during a subsequent catheterization.

Figure 11C shows that the orientation of the contours of the elongated bodily structure are not in alignment with the segmented pre-operative data 24. A control unit is adapted to reconstruct the orientation / trajectory of virtual representations of the elongated bodily structure in the data by comparing the two- or three-dimensional data with the segmented pre-operative data, e.g. using a three-dimensional centerline detection algorithm. The control unit is further adapted to merge the virtual representations of the elongated bodily structure 4 of the two- or three-dimensional data and the segmented pre-operative data to map the access site, a landmark, and/or a characteristic of the elongated bodily structure more accurately and preferably display the locations to a clinician on a display.

The determination of the orientation / trajectory allows to take the patients unique anatomy into consideration and reduces complexity of subsequent surgical intervention, in particular catheterization, by clinicians.

## Claims

**1.** A computer implemented method for determining an access site (1) for an intravascular access for a diagnostic or a surgical intervention, preferably executed on at least one control unit (2), the method comprising the steps:

(a) receiving two- or three-dimensional image data (3) of an elongated bodily structure (4) of a patient transmitted from an imaging probe (5), in particular an ultrasound probe,
(b) positioning the imaging probe (5) by actuation of a positioning module (6) in a first orientation (51) based on the image data (3) such that a two-dimensional imaging plane (53) is aligned essentially orthogonally to a longitudinal axis (L) of the elongated bodily structure (4),
(c) moving the imaging probe (5) by actuation of the positioning module (6) based on the image data (3) in the first orientation (51) along the longitudinal axis (L) of the elongated bodily structure (4), in particular in a proximal direction of the elongated bodily structure (4),
(d) analysing the received two- or three-dimensional image data in view of the presence of a landmark (7) of the elongated bodily structure (4), in particular a bifurcation of the elongated bodily structure (4),
(e) if no landmark (7) is detected repeating the steps (a) - (c),
(f) if a landmark (7) is detected based on the received image data (3) the imaging probe (5) is positioned by actuation of the positioning module (6) based on the image data (3) from the first orientation (51) to a second orientation (52), such that the longitudinal axis (L) of the elongated bodily structure (4) is essentially aligned with the two-dimensional imaging plane (53) in proximity of the landmark (7), in particular at the landmark (7),
(g) determining the access site (1) based on the image data (3), in particular image data (3) in the second orientation (52).

**2.** The method according to claim 1, wherein the method comprises positioning the imaging probe (5) in the first orientation (51) based on the image data (3) by actuation of the positioning module (6), prior to positioning the imaging probe (5) in the second orientation (52), such that the longitudinal axis (L) of the elongated bodily structure (4) is centrally arranged in a field of view of the imaging probe (5) and/or the longitudinal axis (L) of the elongated bodily structure is arranged in a center of rotation of the imaging probe (5) by the positioning module (6).

**3.** The method according to one of the preceding claims, wherein positioning the imaging probe (5) in the second orientation comprises rotating the imaging probe (5) with respect to the elongated bodily structure (4) around an angle between 80° - 100°, in particular between 85° and 95°.

**4.** The method according to one of the preceding claims, wherein positioning the imaging probe (5) in the second orientation comprises rotating the imaging probe (5) with respect to the elongated bodily structure (4) and determining an angular position (54) based on at least one of

(i) the image data (3) detected while rotating, in particular based on the increasing cross-section of the detected elongated bodily structure (4) within the image data while rotating, and
(ii) detecting a characteristic of at least one region of interest (12) of the two- or three-dimensional image data (3), in particular at least one region of interest (12) arranged at least at one outer edge of the two-dimensional image data (3), while rotating to determine the angular position at which the second orientation (52) is reached based on reaching an extremum, in particular a local extremum, of the characteristic.

**5.** A computer implemented method for determining an access site (1) for an intravascular access for a surgical intervention, preferably executed on at least one control unit (2), the method comprising the steps:

(a) receiving first two- or three-dimensional image data (3) of an elongated bodily structure (4) of a patient transmitted from a first imaging probe (5), in particular a first ultrasound probe,
(b) positioning the first imaging probe (5) by actuation of a positioning module (6) such that a first two-dimensional imaging plane (53) of the first imaging probe (5) is aligned essentially orthogonally to a longitudinal axis (L) of the elongated bodily structure (4),
(c) moving the first imaging probe (5) by actuation of the positioning module (6) based on the first image data (3) along the longitudinal axis (L) of the elongated bodily structure (4), in particular in a proximal direction of the elongated bodily structure (4),
(d) analysing the received first two- or three-dimensional image data (3) in view of the presence of a landmark (7) of the elongated bodily structure (4), in particular a bifurcation of the elongated bodily structure (4),
(e) if no landmark (7) is detected repeating the steps (a) - (d),
(f) if a landmark (7) is detected based on the received first image data (3) receiving second two- or three-dimensional image data (30) of a second imaging probe (50), the second imaging probe (50), in particular a second ultrasound probe, being mounted or mountable with a defined spatial relationship with respect to the first imaging probe (5) and
the second imaging probe (50) has a second two-dimensional imaging plane (503) which is arranged at an angle with respect to the first two-dimensional imaging plane, the second two- or three-dimensional image data (30) being received while the longitudinal axis (L) of the elongated bodily structure (4) is essentially aligned with the second two-dimensional imaging plane (503) in proximity of the landmark (7), in particular at the landmark (7),
(g) determining the access site (1), in particular based on the image data (3, 30), in particular based on the second image data (30).

**6.** The method according to one of the preceding claims, wherein the method comprises the step of positioning a contact surface (55) of the imaging probe (5) which is mounted on the positioning module (6) on the skin (10) or mucosa or endothelium or eye cornea of the patient to image the elongated bodily structure (4).

**7.** The method according to claims 6, wherein positioning the contact surface (55) is based on

guidance data detected by a guidance sensor (8), in particular an optical guidance sensor, a capacitive guidance sensor, or an inductive guidance sensor, which is connected to the at least one control unit (2), and/or
a predetermined scanning pattern, in particular a geometric scanning pattern.

**8.** The method according to one of the claims 6 - 7 comprising the steps of

- Receiving force data from a force sensor (9) of the positioning module (6) indicative of an engagement pressure of the contact surface (55) of the imaging probe (5) with the skin (10) or mucosa or endothelium or eye cornea of the patient and
- Positioning the contact surface (55) of the imaging probe (5) on the skin (10) or mucosa or endothelium or eye cornea of the patient for imaging the elongated bodily structure (4) at a specific force value or force value range by actuation of the positioning module (6) based on the force data and preferably the image data (3).

**9.** The method according to one of the claims 6 - 8, wherein the step of receiving at least one of the guidance data, the force data, and the image data (3) indicative of incorrect positioning of the contact surface (55) of the imaging probe (5) with respect to the elongated bodily structure (4), comprises

- re-positioning the contact surface (55) of the imaging probe (5) by actuation of the positioning module (6) based on at least one of the received guidance data, the force data, and the image data (3) to image the elongated bodily structure (4), in particular in the first or second orientation (52).

**10.** The method according to one of the preceding claims, wherein a two-dimensional section of the elongated bodily structure (4), and in particular the landmark (7), is anatomically identified by the control unit (2) based on the image data (3) in real time based on at least one algorithm selected from

- an image segmentation algorithm and
- a machine-learning algorithm, in particular based on a convolutional neural network,

and the method further comprises the step of calculating a confidence map based on the at least one algorithm, in particular additionally based on a scan line integration or a random walk algorithm.

**11.** The method according to one of the preceding claims, comprising the step of carrying out a three-dimensional reconstruction of the elongated bodily structure (4) in real time based on a plurality of the detected two- or three-dimensional image data (3).

**12.** The method according to one of the preceding claims, wherein the positioning module (6) preferably has a mounting section (13) for the imaging probe (5) and the method comprises the step of moving the imaging probe (5) along six degrees of freedom by actuation of the positioning module (6).

**13.** The method according to one of the preceding claims, wherein the access site (1) is determined, such that a medical device for insertion into the elongated bodily structure (4) is insertable through skin (10) or mucosa or endothelium or eye cornea at the access site (1) to enter the elongated bodily structure (4), in particular a lumen of the elongated bodily structure (4), wherein the access site () is determined in a range between 1 cm and 6 cm proximally from the landmark (7), in particular in a range between 1.5 cm and 4 cm proximally from the landmark (7).

**14.** A robotic system (101) comprising a control unit (2), a positioning module (6), and in particular an imaging probe (5) mountable on the positioning module (6), having means adapted to execute the steps of the computer implemented method of one of the preceding claims.

**15.** Computer program product comprising instructions that cause the robotic system (101) of claim 14 to perform the method steps of one of the claims 1 - 13.

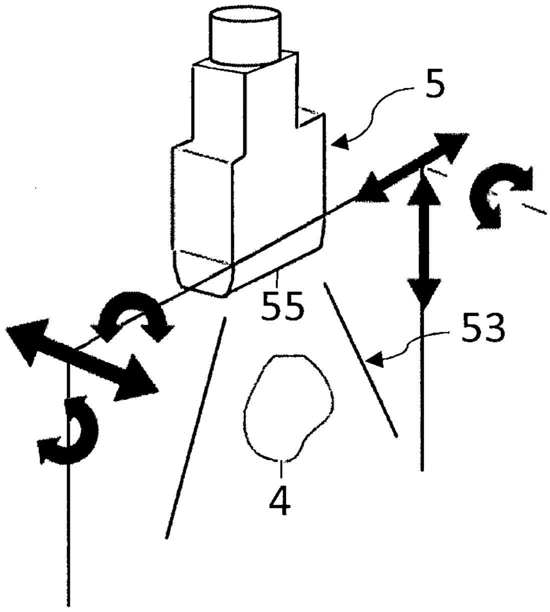


Fig. 1A

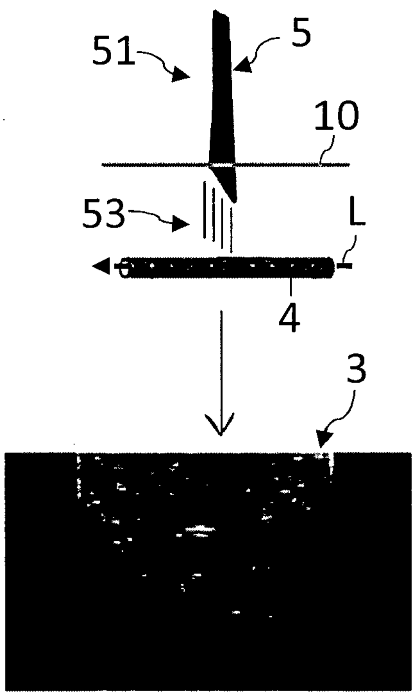


Fig. 1B

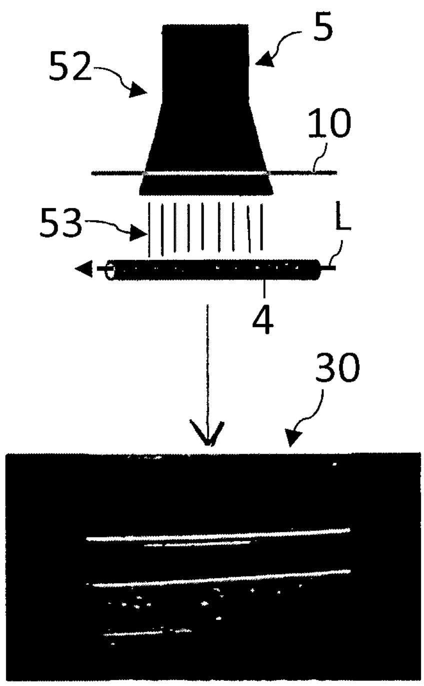


Fig. 1C

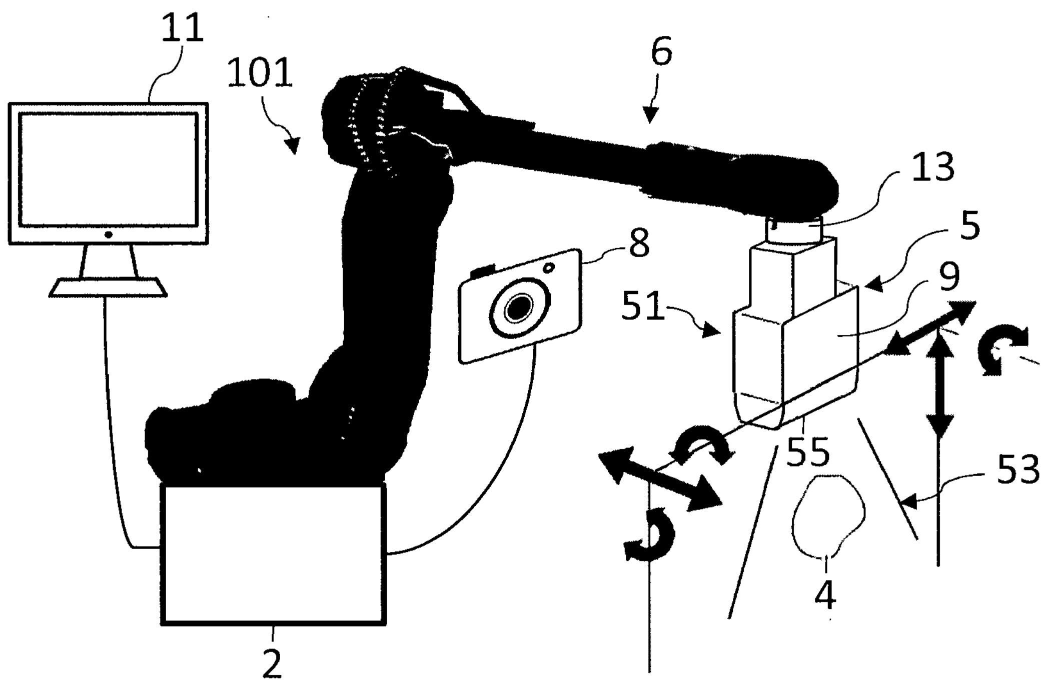


Fig. 2

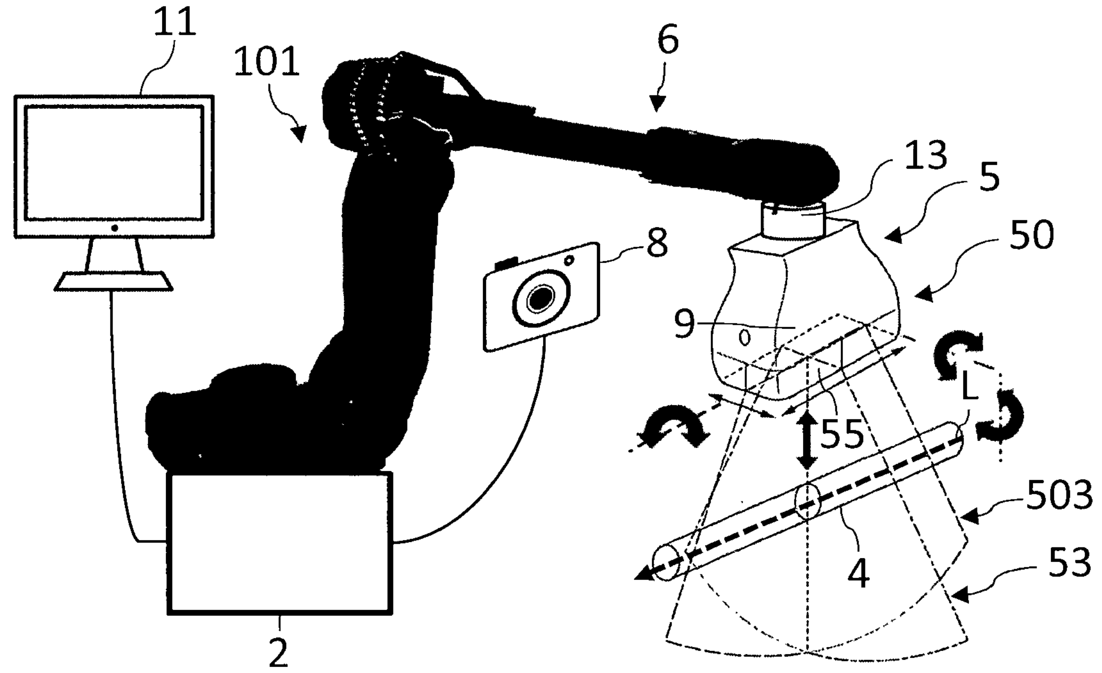


Fig. 3

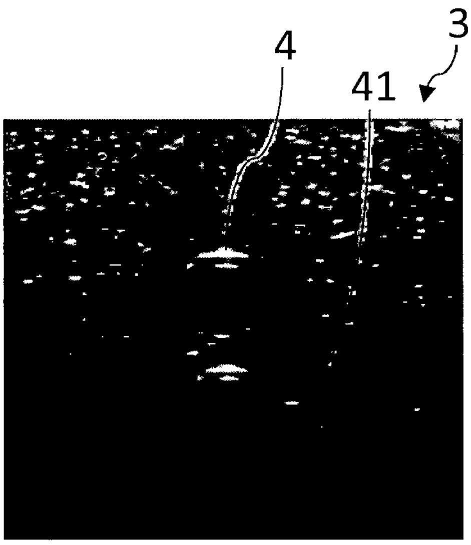


Fig. 4A

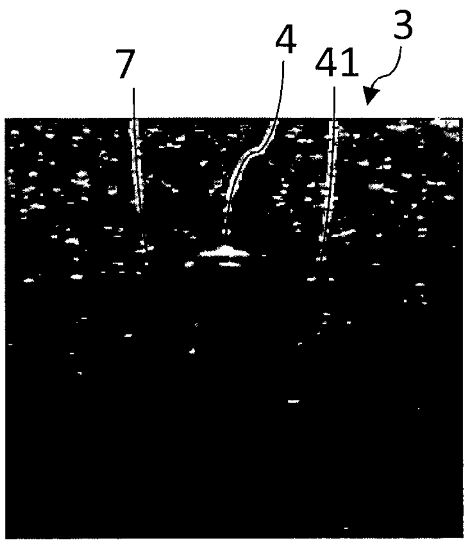


Fig. 4B

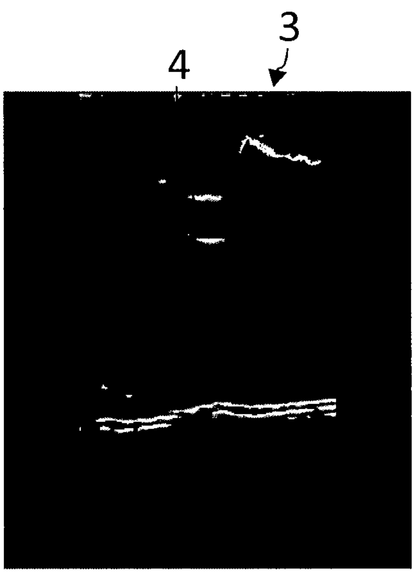


Fig. 4C

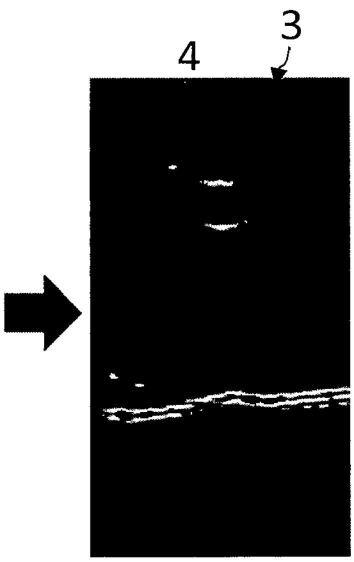


Fig. 4D

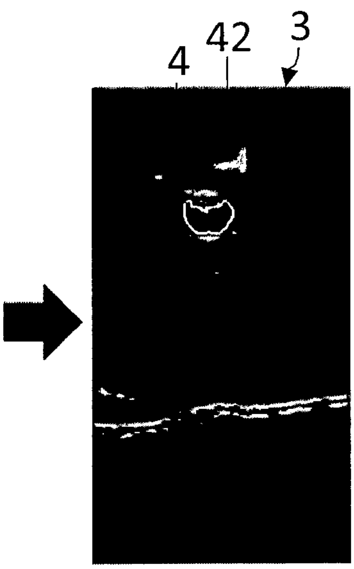


Fig. 4E

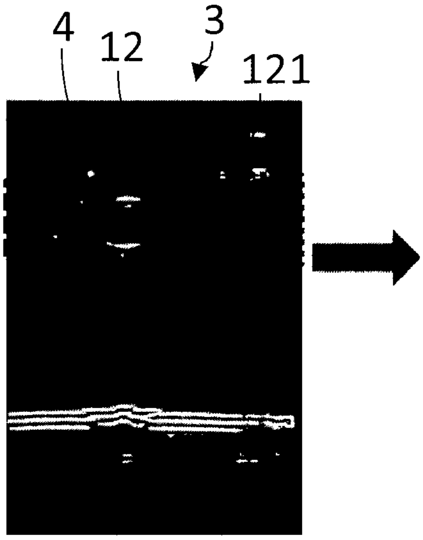


Fig. 5A

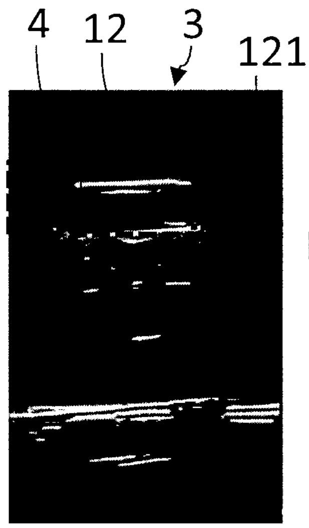


Fig. 5B

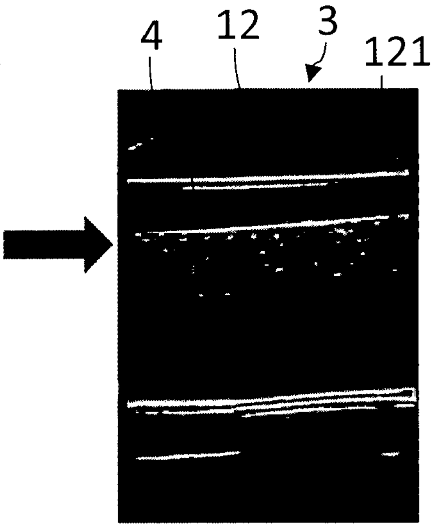


Fig. 5C

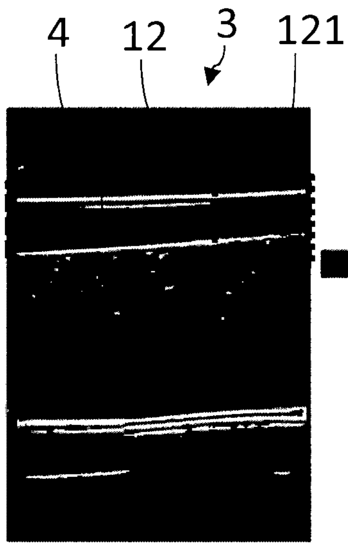


Fig. 5D

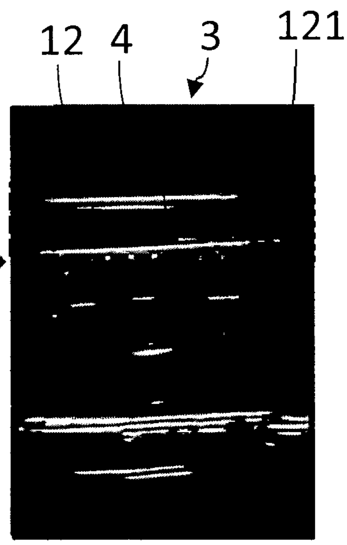


Fig. 5E

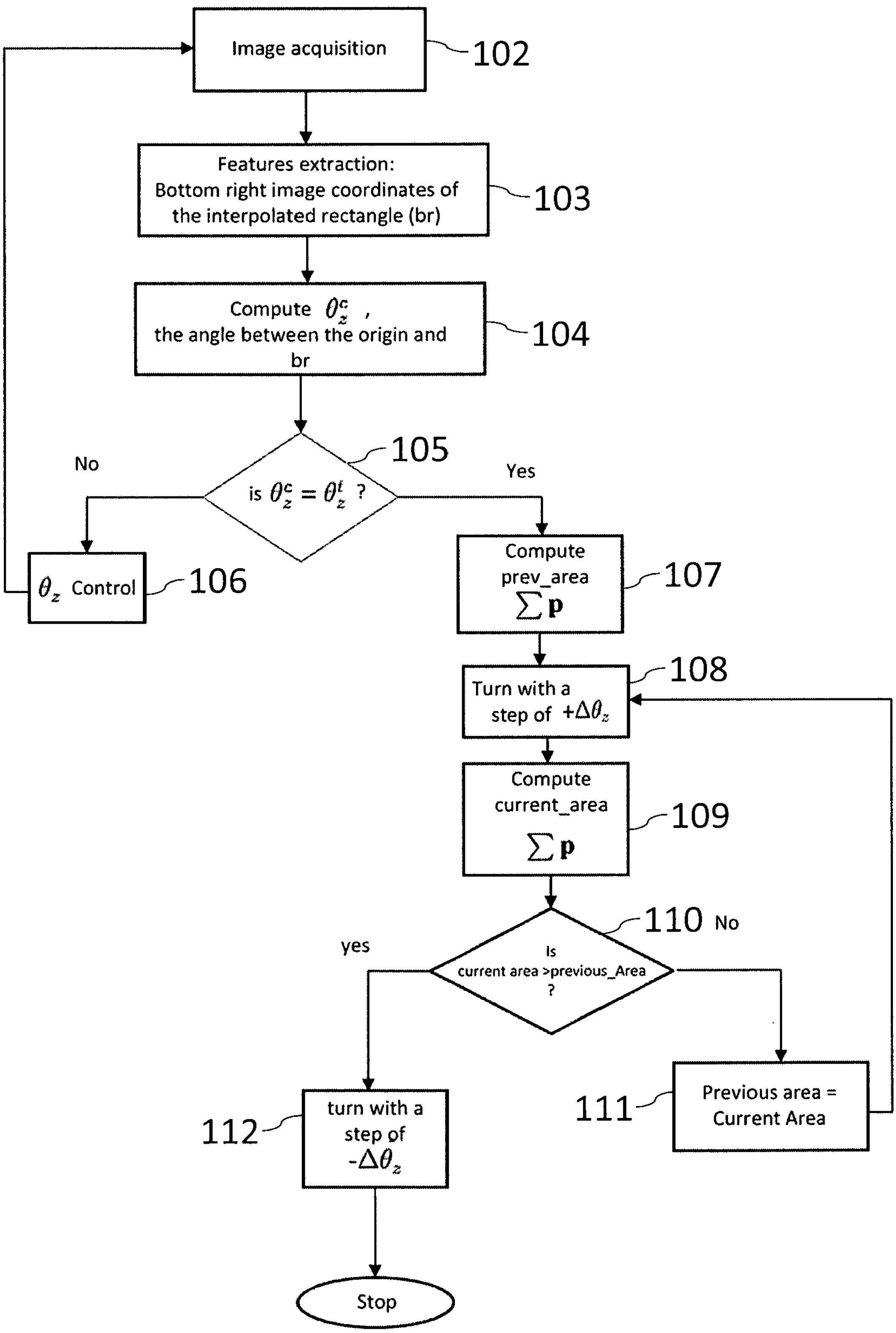
Image acquisition
102
Features extraction:
Bottom right image coordinates of the interpolated rectangle (br)
103
Compute $\theta_z^c$ ,
the angle between the origin and br
104
105
No
Yes
is $\theta_z^c = \theta_z^t$ ?
$\theta_z$ Control
106
Compute prev_area
$\sum \mathbf{p}$
107
108
Turn with a step of $+\Delta\theta_z$
Compute current_area
$\sum \mathbf{p}$
109
110
No
yes
Is current area >previous_Area ?
111
Previous area = Current Area
112
turn with a step of $-\Delta\theta_z$
Stop

Fig. 5F

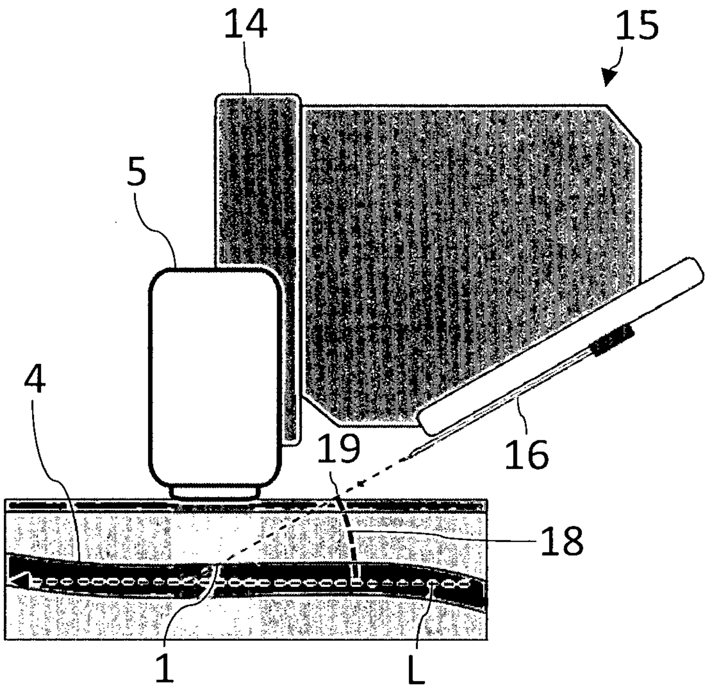


Fig. 6A

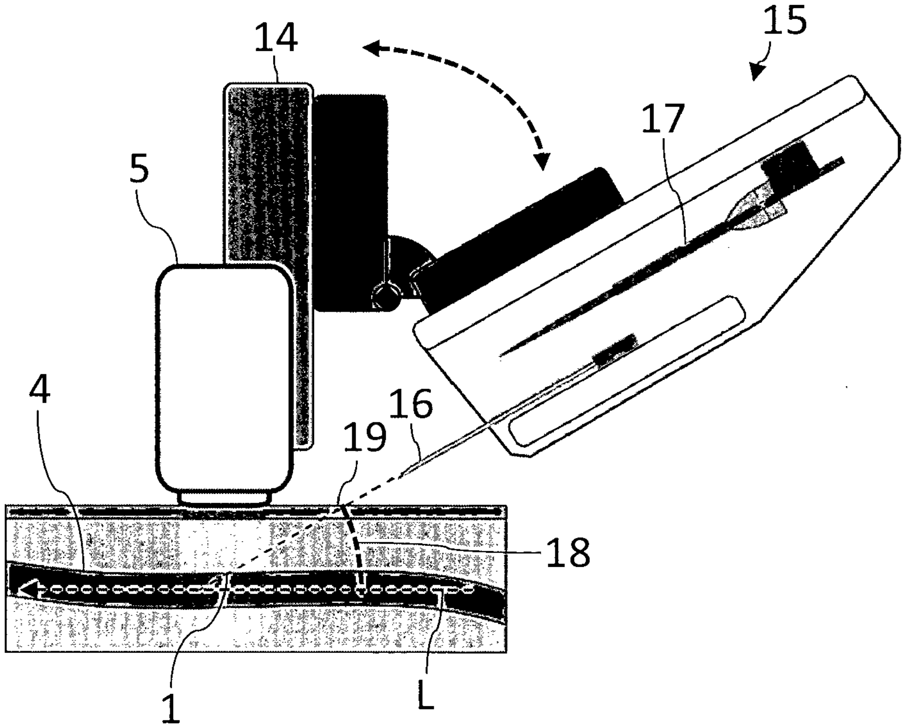


Fig. 6B

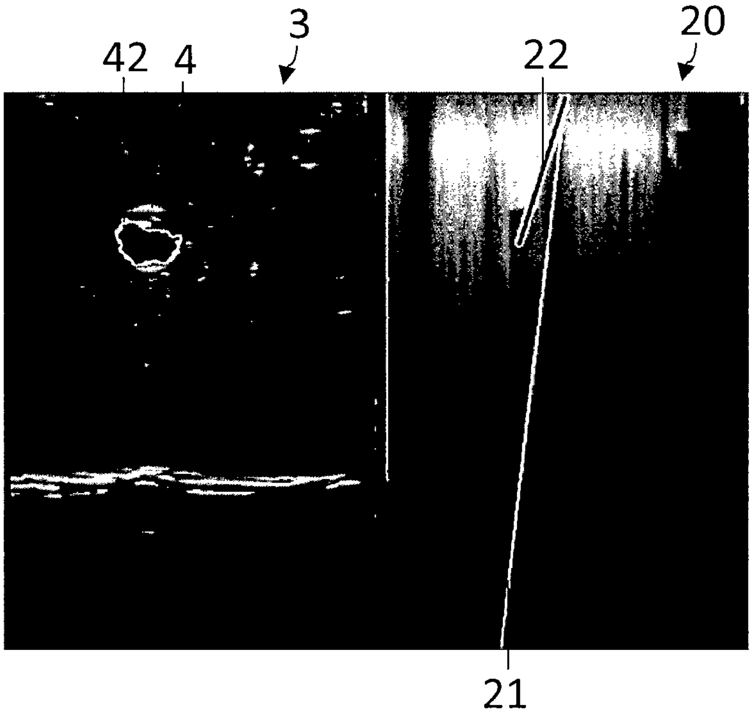


Fig. 7A

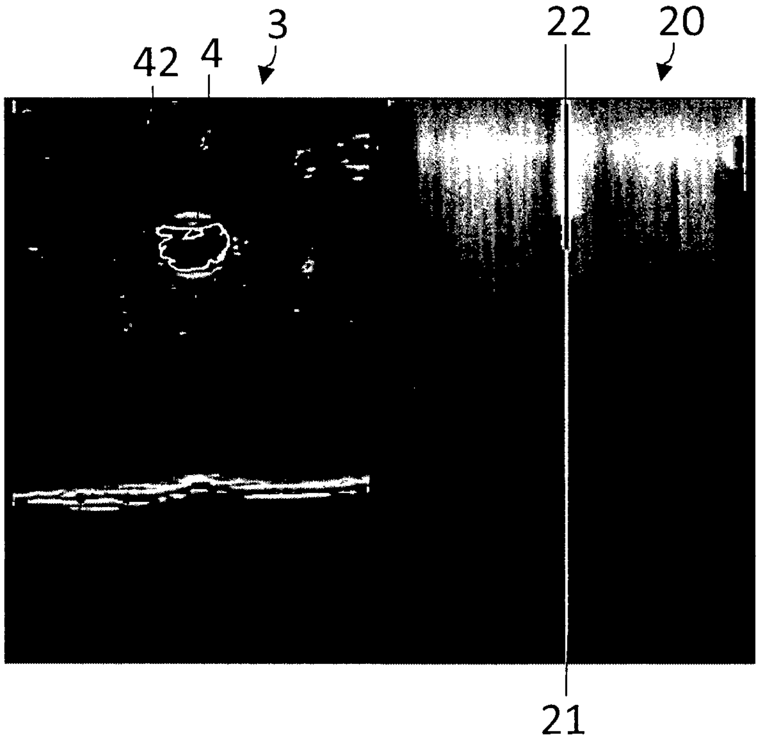


Fig. 7B

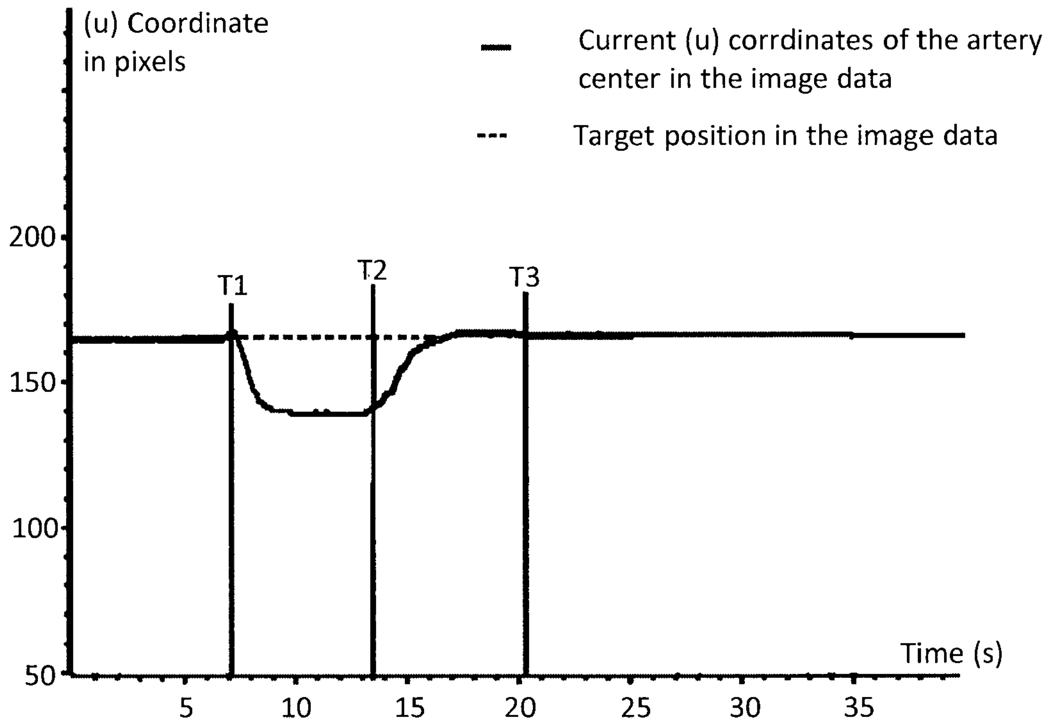
(u) Coordinate
in pixels
Current (u) corrdinates of the artery
center in the image data
Target position in the image data
200
150
100
50
T1
T2
T3
Time (s)
5
10
15
20
25
30
35

**Fig. 8**

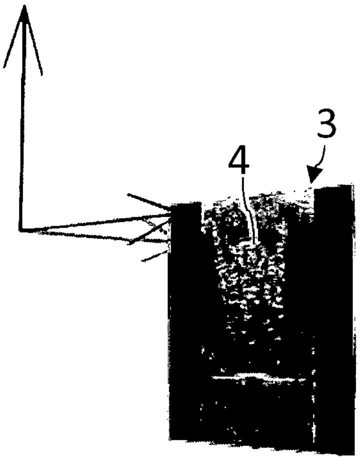


Fig. 9A

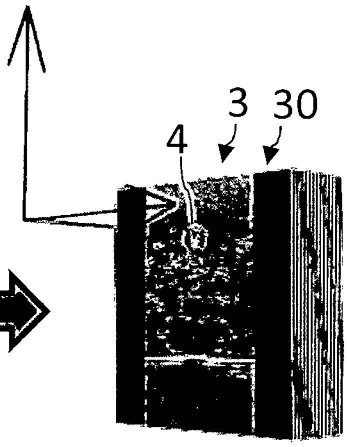


Fig. 9B

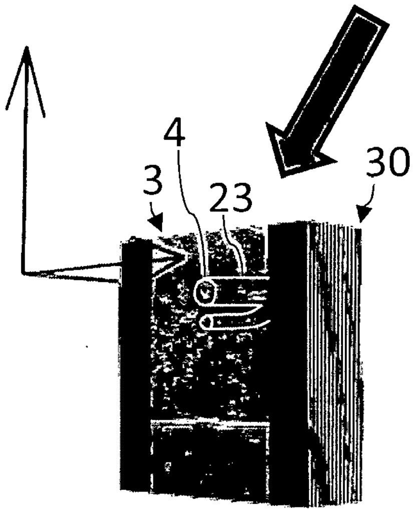


Fig. 9C

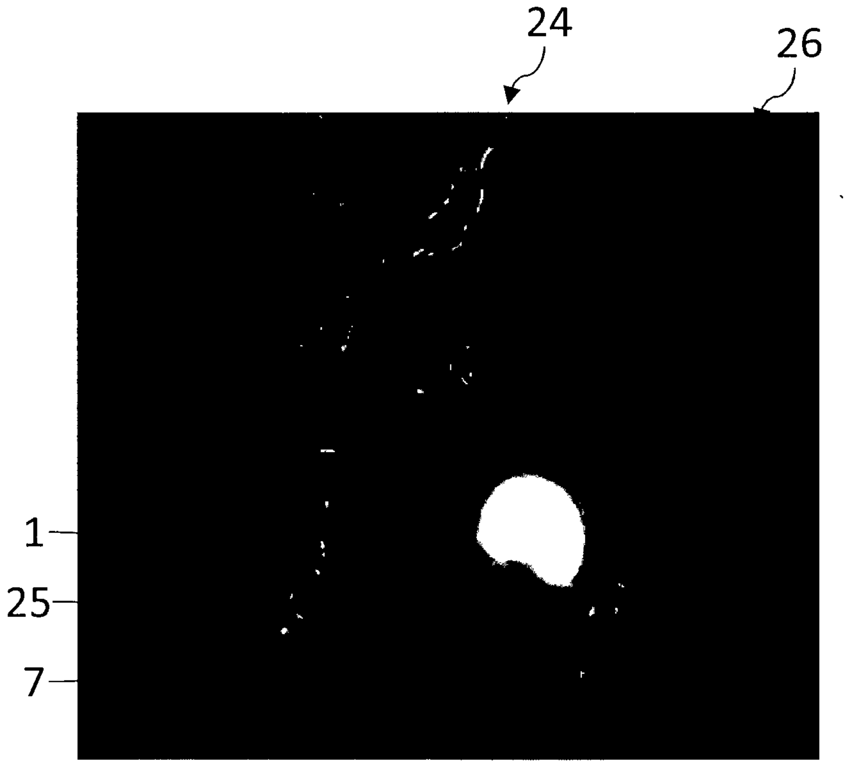


Fig. 10A

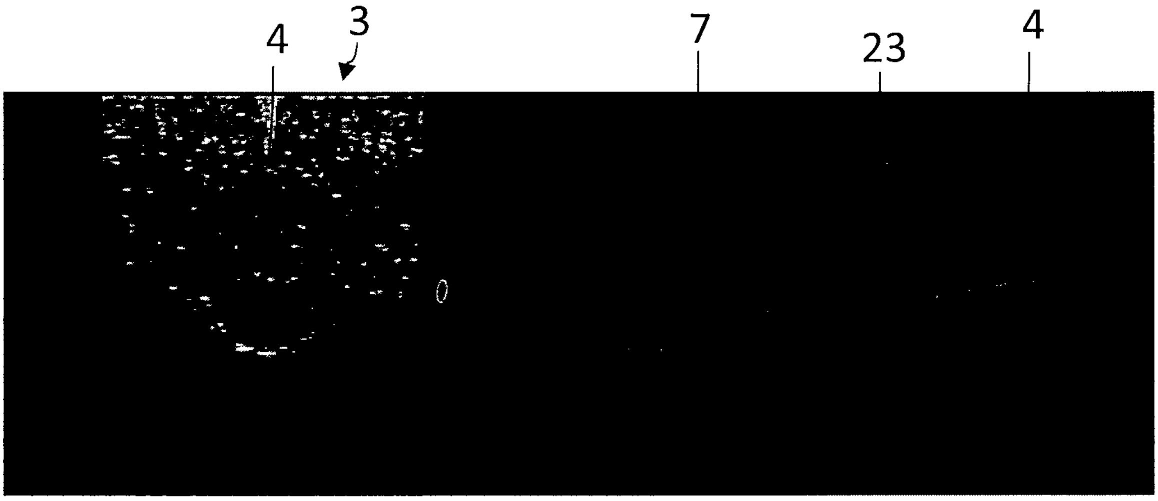


Fig. 11A

Fig. 11B

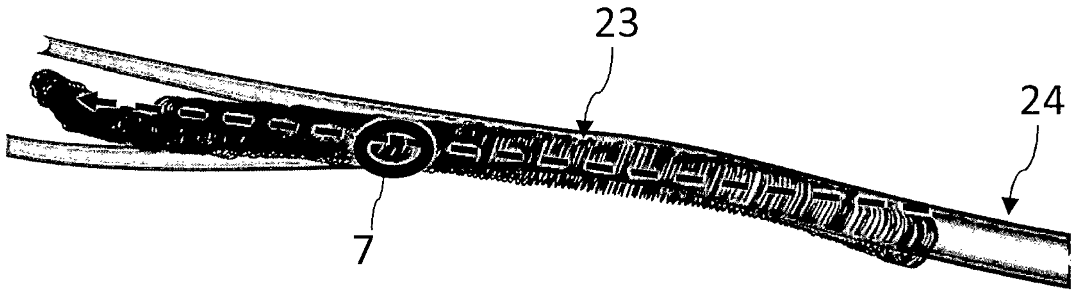


Fig. 11C

Europäisches Patentamt
European Patent Office
Office européen des brevets

# EUROPEAN SEARCH REPORT

Application Number
EP 23 31 5279

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 4 104 768 A1 (ASAHI INTECC CO LTD [JP]; FUJI CORP [JP]) 21 December 2022 (2022-12-21) * paragraphs [0003], [0006], [0032] - [0036], [0051], [0061], [0085], [0093] - [0107], [0177] - [0185]; figures 1-3,12-13,22-26 * ----- | 1-15 | INV. A61B8/08 A61B8/00 |
| Y | XENOGIANNIS IOSIF ET AL: "Ultrasound-Guided Femoral Vascular Access for Percutaneous Coronary and Structural Interventions", DIAGNOSTICS, vol. 13, no. 12, 11 June 2023 (2023-06-11), page 2028, XP093113865, CH ISSN: 2075-4418, DOI: 10.3390/diagnostics13122028 Retrieved from the Internet: URL:https://www.ncbi.nlm.nih.gov/pmc/articles/PMC10297166/pdf/diagnostics-13-02028.pdf> * abstract; figures 1,2 * * page 5, paragraph 3 - page 7, paragraph 1 * ----- | 1-15 | TECHNICAL FIELDS SEARCHED (IPC): A61B |
| A | KR 2018 0027056 A (UNIV KEIMYUNG IACF [KR]) 14 March 2018 (2018-03-14) * figures 3-5 * ----- -/-- | 5 | |

The present search report has been drawn up for all claims

| Place of search | Date of completion of the search | Examiner |
|---|---|---|
| Munich | 21 December 2023 | Daoukou, Eleni |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 23 31 5279

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZEVALLOS NICO ET AL: "Toward Robotically Automated Femoral Vascular Access", 2021 INTERNATIONAL SYMPOSIUM ON MEDICAL ROBOTICS (ISMR), IEEE, 17 November 2021 (2021-11-17), pages 1-7, XP033996895, DOI: 10.1109/ISMR48346.2021.9661560 [retrieved on 2021-12-22] * page 3, left-hand column, paragraph 4 - page 4, left-hand column, paragraph 2; figures 1,3 * ----- | 7-9 | |
| A | US 2011/166451 A1 (BLAIVAS MICHAEL [US] ET AL) 7 July 2011 (2011-07-07) * figures 1,2c,2e * ----- | 11 | |
| A | ZHONGLIANG JIANG ET AL: "Precise Repositioning of Robotic Ultrasound: Improving Registration-based Motion Compensation using Ultrasound Confidence Optimization", ARXIV.ORG, CORNELL UNIVERSITY LIBRARY, 201 OLIN LIBRARY CORNELL UNIVERSITY ITHACA, NY 14853, 10 August 2022 (2022-08-10), XP091291814, * abstract; figure 2 * ----- | 10 | TECHNICAL FIELDS SEARCHED (IPC) |

The present search report has been drawn up for all claims

| Place of search | Date of completion of the search | Examiner |
|---|---|---|
| Munich | 21 December 2023 | Daoukou, Eleni |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

# ANNEX TO THE EUROPEAN SEARCH REPORT ON EUROPEAN PATENT APPLICATION NO.

EP 23 31 5279

This annex lists the patent family members relating to the patent documents cited in the above-mentioned European search report. The members are as contained in the European Patent Office EDP file on
The European Patent Office is in no way liable for these particulars which are merely given for the purpose of information.

21-12-2023

| Patent document cited in search report | Publication date | Patent family member(s) | Publication date |
|---|---|---|---|
| EP 4104768 A1 | 21-12-2022 | EP 4104768 A1 | 21-12-2022 |
| | | JP WO2021161516 A1 | 19-08-2021 |
| | | US 2022378396 A1 | 01-12-2022 |
| | | WO 2021161516 A1 | 19-08-2021 |
| KR 20180027056 A | 14-03-2018 | NONE | |
| US 2011166451 A1 | 07-07-2011 | CA 2786298 A1 | 14-07-2011 |
| | | EP 2521498 A1 | 14-11-2012 |
| | | JP 2013516288 A | 13-05-2013 |
| | | US 2011166451 A1 | 07-07-2011 |
| | | US 2012172722 A1 | 05-07-2012 |
| | | US 2013150724 A1 | 13-06-2013 |
| | | WO 2011085135 A1 | 14-07-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021161516 A1
- US 2003167005 A1